# FASCICULE DE BREVET EUROPEEN

(11) **EP 4 188 076 B1**
(45) Date de publication et mention de la délivrance du brevet: **25.06.2025**
(21) Numéro de dépôt: 21756020.0
(22) Date de dépôt: 27.07.2021
(51) Int. Cl.: A01H 1/02, F04F 5/46, F04F 5/54

(54) **AMPLIFICATEUR DE DEBIT A EFFET COANDA ET DISPOSITIF AERAULIQUE COMPRENANT UN TEL AMPLIFICATEUR DE DEBIT**
COANDA-EFFEKT-DURCHFLUSSVERSTÄRKER UND LUFTEINRICHTUNG BEINHALTEND EINEN SOLCHEN DURCHFLUSSVERSTÄRKER
COANDA EFFECT FLOW AMPLIFIER AND AERAULIC DEVICE INCLUDING SUCH FLOW AMPLIFIER

(30) Priorité: 29.07.2020 FR 2008012
(43) Date de publication de la demande: 07.06.2023
(73) Titulaire: Institut national de recherche pour l'agriculture, l'alimentation et l'environnement, 75007 Paris (FR); Syngenta Crop Protection AG, 4058 Basel (CH); ASUR PLANT BREEDING, 60190 Estrées-Saint-Denis (FR)
(72) Inventeur: BALDET, Patrick, 45290 PRESSIGNY LES PINS (FR)
(74) Mandataire: Ipsilon
(86) Numéro de dépôt international: PCT/FR2021/051400
(87) Numéro de publication internationale: WO 2022/023663

(56) Documents cités:
- DE-A1- 102009 047 089
- FR-A1- 3 078 859
- GB-A- 2 169 192
- GB-A- 2 234 782

## Description

La présente invention concerne le domaine de l'amplification de débit de flux de gaz.

En particulier, l'invention concerne un amplificateur de débit à effet Coanda. Les amplificateurs de débit d'air à effet Coanda sont également nommés dans les pays anglo-saxons « Air Amplifier » ou « Air Mover ». L'invention concerne également un dispositif aéraulique comprenant un tel amplificateur de débit à effet Coanda.

Un amplificateur de débit à effet Coanda comprend notamment un conduit principal de circulation d'un fluide, par exemple de l'air. L'amplificateur de débit permet d'induire un flux d'aspiration en amont d'un conduit principal et un flux de soufflage en aval de ce conduit principal. Ce flux de soufflage est constitué d'un flux moteur primaire injecté sous pression dans le conduit principal et d'un flux secondaire induit par l'effet Coanda. En particulier, un profil spécifique du conduit principal rencontré par le flux moteur primaire permet de générer un effet Coanda de manière à induire le flux secondaire. Cet effet Coanda permet d'obtenir un effet multiplicateur entre le flux secondaire induit et le flux moteur primaire très important, notamment en comparaison avec un générateur de débit d'air à effet Venturi. Le flux moteur primaire est généralement de l'air comprimé.

Il existe un grand nombre d'amplificateurs de débit d'air à effet Coanda conçus pour l'aspiration de gaz propres ou chargés de particules destinés à être évacués en qualité de déchets et donc sans considération aucune de l'impact de ces dispositifs d'aspiration sur le maintien de l'intégrité de particules très fragiles. Ces appareils du commerce, mus par énergie pneumatique, ont été conçus pour des ambiances présentant des risques d'inflammation dans lesquelles les aspirateurs mécaniques peuvent engendrer frottements, chaleur et étincelles. Ces amplificateurs de débit d'air à usage industriels sont en particulier utilisés pour dégazer les cales des bateaux marchands et fonctionnent à des pressions d'air ou de vapeur pouvant atteindre 8 bars de pression relative et induire des vitesses de flux d'air d'aspiration supérieures à 50 mètres par seconde (180 km/h). Les profils intérieurs utilisés qui génèrent une dépression par effet Coanda ont été optimisés pour ces valeurs élevées de pression et de vitesses de fonctionnement, la forme de ces profils s'apparente à celle des extrados d'ailes d'avions rapides avec pour corollaire des plages de vitesses de fonctionnement optimal assez réduites. Il est connu du document GB-2234782 une buse à effet Coanda aux capacités d'aspiration variables.

Le principe de fonctionnement d'un amplificateur de débit à effet Coanda le rend particulièrement prédisposé au convoyage de particules très fragiles. En effet, un amplificateur de débit à effet Coanda a pour avantage majeur de présenter un minimum d'obstacles au niveau de la section de passage du conduit principal, notamment en comparaison à des systèmes d'amplification comprenant un ventilateur dans le conduit de passage ou de type Venturi dont l'architecture prévoit une réduction importante du diamètre interne de passage de l'air ainsi que la présence dans le conduit principal d'une ou plusieurs buses d'injection d'air primaire.

Le pollen est un exemple de matière fragile pouvant être convoyée en utilisant un amplificateur de débit à effet Coanda. En particulier, les pollens dits « Récalcitrants », par analogie à la classification des semences, sont destinés à une pollinisation quasi-immédiate car leur viabilité est très réduite dans le temps et conditionnée au maintien d'un niveau élevé d'hydratation. C'est le cas des pollens de poacées comme celui des blés (triticum sp.) des orges (hordeum sp.), du riz (oryza sp.) ou encore du maïs (zea mays sp.). Ces pollens ne peuvent pas être facilement conservés, ils sont très fragiles et requièrent beaucoup de précautions pour leur manipulation. La pollinisation artificielle des plantes dotées de tels pollens implique des technologies et pratiques spécifiques respectueuses de la viabilité très éphémère de ces pollens. La viabilité du pollen correspond à son potentiel reproducteur. Il est ainsi particulièrement pertinent d'utiliser un amplificateur de débit à effet Coanda pour convoyer ce type de pollen. Le document FR-3078859 enseigne un dispositif aéraulique à effet Coanda pour la pollinisation.

L'utilisation des amplificateurs de débit à effet Coanda connus à des vitesses d'aspiration de moins de 10m.s-1 demeure possible mais lesdites vitesses se trouvent en deçà des plages optimales de fonctionnement. Les performances de l'amplificateur de débit sont ainsi très modestes. De plus, les entrées d'air moteur des amplificateurs de débit d'air connus sont conçues pour des pressions de fonctionnement élevées avec une optimisation limitée des pertes de charge, elles sont de surcroît fréquemment tangentielles à l'anneau interne de répartition de l'air moteur sur le profil Coanda ; il en résulte des tourbillons internes indésirables de nature à projeter par centrifugation les particules aspirées sur les parois. En effet, des tourbillons peuvent intervenir très facilement dans un flux basse pression.

En outre, il a été observé que l'uniformité et la stabilité du flux de gaz moteur injecté dans le conduit principal avec ces amplificateurs de débit d'air connus ne peuvent être garanties avec précision sur la totalité de la section de passage de l'air. Ceci peut provoquer des turbulences dans le flux de soufflage pouvant endommager les matières fragiles transportées qui requièrent en effet un flux de soufflage uniforme et maitrisé.

Les amplificateurs de débit d'air connus sont également peu optimisés en termes de masse et d'encombrement. Ces amplificateurs de débit d'air peuvent ainsi être difficiles à intégrer au sein de systèmes aérauliques ou induire des contraintes importantes d'installation.

Il existe donc un besoin pour un amplificateur de débit à effet Coanda ne présentant pas les inconvénients précités. En particulier, il existe un besoin pour un amplificateur de débit à effet Coanda dont les performances aérauliques sont améliorées pour des faibles vitesses de convoyage, par exemple inférieures à 10m.s-1, de manière à permettre le convoyage de matières fragiles.

Pour cela, l'invention concerne un amplificateur de débit à effet Coanda pour induire un flux d'air amplifié, selon la revendication 1. Il comporte :
- un conduit principal de circulation d'air,
   au moins un orifice d'injection débouchant dans le conduit principal,
- une pluralité d'orifices d'alimentation en gaz moteur comprimé chacun configuré pour être connecté à une source de gaz moteur comprimé pour alimenter ledit au moins un orifice d'injection en gaz moteur comprimé,
- au moins un conduit de distribution reliant ladite pluralité d'orifices
   d'alimentation audit au moins un orifice d'injection,
- un profil d'amplification délimitant au moins partiellement ledit au moins un orifice d'injection et formant une surface convexe configurée pour induire un effet Coanda sur un flux de gaz moteur comprimé injecté au travers dudit au moins un orifice d'injection.

L'utilisation d'un amplificateur de débit à effet Coanda ayant une pluralité de d'orifices d'alimentation permet une répartition améliorée du gaz moteur à l'intérieur du conduit de distribution et au travers du ou des orifices d'injections. Le flux de gaz moteur injecté à l'intérieur du conduit principal est ainsi plus uniforme et plus stable que dans une configuration connue d'amplificateur de débit à effet Coanda ne comportant qu'un seul orifice d'alimentation. Par exemple, la présence d'au moins deux orifices d'alimentation de l'amplificateur permet un gain important, d'au moins 25%, en termes de débit d'air comprimé dans l'amplificateur, ainsi qu'aspiré et soufflé dans le conduit principal.

Les amplificateurs de débit d'air à effet Coanda connus ont généralement un conduit de distribution sous la forme d'une cavité annulaire. L'alimentation de cette cavité annulaire en gaz moteur a tendance à générer des tourbillons de par la géométrie de cette cavité. L'utilisation d'une pluralité d'orifices d'alimentation permet de mélanger les flux de gaz moteur et de les amortir afin d'alimenter le ou les orifices d'injection avec un flux de gaz moteur le plus uniforme possible.

On entend par « uniforme », un flux le plus laminaire possible. En particulier, la vitesse du gaz moteur au travers de la pluralité d'orifices d'injection est sensiblement identique à un instant donné. On entend par « sensiblement identique » le fait que ces vitesses de gaz moteur sont comprises dans un intervalle inférieur ou égal à 2 m.s-1, de préférence inférieur ou égal à 1 m.s-1. Ce caractère uniforme s'entend pour un conduit de distribution donné. Dans l'hypothèse où l'amplificateur de débit comprend une pluralité de conduits de distribution, chaque conduit de distribution présente un flux de gaz moteur uniforme.

De plus, cette configuration dans laquelle l'amplificateur de débit comprend une pluralité d'orifices d'alimentation permet d'envisager une segmentation de l'alimentation en gaz moteur de manière à obtenir des vitesses volontairement différentes selon les orifices d'injection.

Selon l'invention, le conduit principal de circulation d'air s'étend le long d'un axe de circulation, ledit au moins un conduit de distribution formant une cavité de distribution annulaire s'étendant le long et autour de l'axe de circulation, ledit au moins un orifice d'injection formant une fente s'étendant au moins partiellement autour de l'axe de circulation.

Selon l'invention, une dimension radiale de la fente est limitée par la présence d'un rayon de raccordement présentée par une paroi de déflexion faisant face à chacun des orifices d'alimentation, cette paroi de déflexion étant adjacente à la fente débouchant sur l'orifice d'injection.

Cette fente peut être formée de manière continue en une seule fente ou de manière discontinue avec une pluralité de portions de fente. Il est ainsi possible de former une fente segmentée par la suite par des éléments de cloison afin d'obtenir ces portions de fente.

Selon un mode de réalisation de l'amplificateur de débit, ledit au moins un orifice d'injection est formé par une cavité d'injection annulaire s'étendant autour de l'axe de circulation et radialement par rapport à cet axe de circulation.

La cavité d'injection forme ainsi un disque dont l'extrémité interne débouche à l'intérieur du conduit principal et dont l'extrémité opposée communique avec ledit au moins un conduit de distribution.

Selon un mode de réalisation de l'amplificateur de débit, les orifices d'alimentation sont orientés transversalement à l'axe de circulation, l'amplificateur de débit comprenant en outre au moins une paroi de déflexion faisant face à chacun des orifices d'alimentation.

L'orientation transversale des orifices d'alimentation combinée à la présence d'une paroi de déflexion permet d'amortir le flux de gaz moteur alimentant le conduit de distribution. Cet amortissement permet de stabiliser le flux de gaz moteur avant d'atteindre la pluralité d'orifices d'injection. Selon une configuration particulière, les orifices d'alimentation sont orientés radialement à l'axe de circulation.

Selon un mode de réalisation de l'amplificateur de débit, celui-ci comprend une pluralité de conduits de distribution indépendants les uns par rapport aux autres et une pluralité d'orifices d'injection, chaque conduit de distribution s'étendant entre au moins l'un parmi la pluralité d'orifices d'alimentation et au moins l'un parmi la pluralité d'orifices d'injection de manière à pouvoir injecter au travers de la pluralité d'orifices d'injection des flux de gaz moteur comprimé distincts.

L'indépendance des conduits de distribution permet de former des lignes de distribution distinctes débouchant au niveau d'orifices d'injection distincts. Il est ainsi possible d'injecter des flux de gaz moteur distincts dont les propriétés physiques ou physico-chimiques sont différentes. En effet, il est possible d'injecter des flux de gaz moteur de vitesses différentes ou de natures de gaz différentes.

Selon un mode de réalisation de l'amplificateur de débit, ladite pluralité de conduits de distribution est formée par la cavité de distribution annulaire, l'amplificateur de débit comprenant en outre au moins deux éléments de cloison permettant de compartimenter la cavité de distribution pour former au moins deux conduits de distribution indépendants.

Selon un mode de réalisation de l'amplificateur de débit, celui-ci comprend en outre des moyens de réglage d'une section de passage du gaz moteur dudit au moins un orifice d'injection de manière à réguler le débit de gaz moteur traversant in fine ledit au moins un orifice d'injection.

Selon un mode de réalisation de l'amplificateur de débit, les moyens de réglage sont configurés pour régler distinctement la section de passage de gaz moteur d'au moins deux orifices d'injection communiquant avec des conduits de distribution indépendants de manière à pouvoir injecter au travers desdits au moins deux orifices des flux de gaz moteur comprimé de débits différents.

Selon un mode de réalisation de l'amplificateur de débit, la pluralité d'orifices d'injection comprend au moins un premier et au moins un deuxième orifices d'injection destinés à être disposés respectivement en partie inférieure et en partie supérieure du conduit principal de circulation d'air de manière à pouvoir induire en parties inférieur et supérieure un débit d'air secondaire amplifié différent.

Selon un mode de réalisation de l'amplificateur de débit, les moyens de réglage sont configurés pour régler distinctement la section de passage de gaz moteur d'au moins quatre orifices d'injection communiquant avec des conduits de distribution indépendants, la pluralité d'orifices d'injection comprenant en outre au moins un troisième et au moins un quatrième orifices d'injection destinés à être disposés respectivement au niveau de parties latérales opposées du conduit principal de circulation d'air.

Selon un mode de réalisation de l'amplificateur de débit, celui-ci comprend :
- un corps dans lequel sont formés ledit conduit principal de circulation d'air, la pluralité d'orifices d'alimentation, ledit au moins un conduit de distribution, le profil d'amplification et une première portion dudit orifice d'injection,
- une couronne d'injection formant une deuxième portion dudit au moins un orifice d'injection, la couronne d'injection étant configurée pour être disposée en regard du corps, les première et deuxième portions dudit au moins un orifice d'injection se faisant face, la distance séparant les première et deuxième portions dudit au moins un orifice d'injection définissant une section de passage de gaz moteur au travers dudit au moins un orifice d'injection.

Selon un mode de réalisation de l'amplificateur de débit, les moyens de réglage sont configurés pour régler la distance entre la couronne d'injection et le corps de manière à faire varier la section de passage du gaz moteur dudit au moins un orifice d'injection.

Selon un mode de réalisation de l'amplificateur de débit, la couronne est mobile par rapport au corps autour d'au moins un axe transversal à un axe de circulation du conduit principal de circulation, les moyens de réglage étant configurés pour régler l'angle d'inclinaison de la couronne par rapport audit au moins un axe transversal de manière à faire varier la section de passage de gaz moteur dudit au moins un orifice d'injection de manière asymétrique.

L'invention concerne également un appareil aéraulique pour la pollinisation d'au moins une plante receveuse à partir du pollen capté sur au moins une plante donneuse, comprenant :
- un organe de captation du pollen depuis ladite au moins une plante donneuse,
- au moins un organe de diffusion du pollen sur au moins une plante receveuse,
- un canal de convoyage du pollen capté depuis l'organe de captation vers le ou les organes de diffusion, et
- au moins un amplificateur de débit tel que décrit ci-avant.

L'invention concerne également une utilisation d'un amplificateur de débit tel que décrit ci-avant pour l'amplification d'un flux d'air comprenant des particules présentant une vitesse de sédimentation prédéterminée, dans laquelle l'amplificateur de débit à effet Coanda induit un flux d'air à l'intérieur du conduit principal de circulation dont la vitesse est supérieure à la vitesse de sédimentation prédéterminée.

Selon un mode de réalisation de l'utilisation de l'amplificateur de débit, la vitesse du flux d'air induit à l'intérieur du conduit principal de circulation est égale ou inférieure à 10m.s-1, de préférence égale ou inférieure à 5m.s-1.

### Brève description des dessins

Les dessins annexés illustrent l'invention :
[Fig. 1] représente une vue en perspective d'un amplificateur de débit à effet Coanda selon l'invention.
[Fig. 2] représente une vue schématique en coupe d'un schéma de principe de l'amplificateur de débit à effet Coanda de la figure 1, dans lequel un flux d'aspiration, un flux de soufflage et un flux de gaz moteur sont illustrés.
[Fig. 3] représente une vue en coupe transversale de l'amplificateur de débit de la figure 1.
[Fig. 4] représente une vue détaillée de la figure 3.
[Fig. 5] représente une vue en perspective d'un corps principal de l'amplificateur de débit de la figure 1.
[Fig. 6] représente une vue éclatée de l'amplificateur de débit de la figure 1.
[Fig. 7] représente un set de deux éléments réducteurs de section du conduit de distribution en air moteur primaire appartenant à un kit d'amplification comprenant également l'amplificateur de débit de la figure 1.
[Fig. 8a] et [Fig. 8b] représentent des vues détaillées de variantes de réalisation de la vue détaillée de la figure 4.

### Description de mode(s) de réalisation

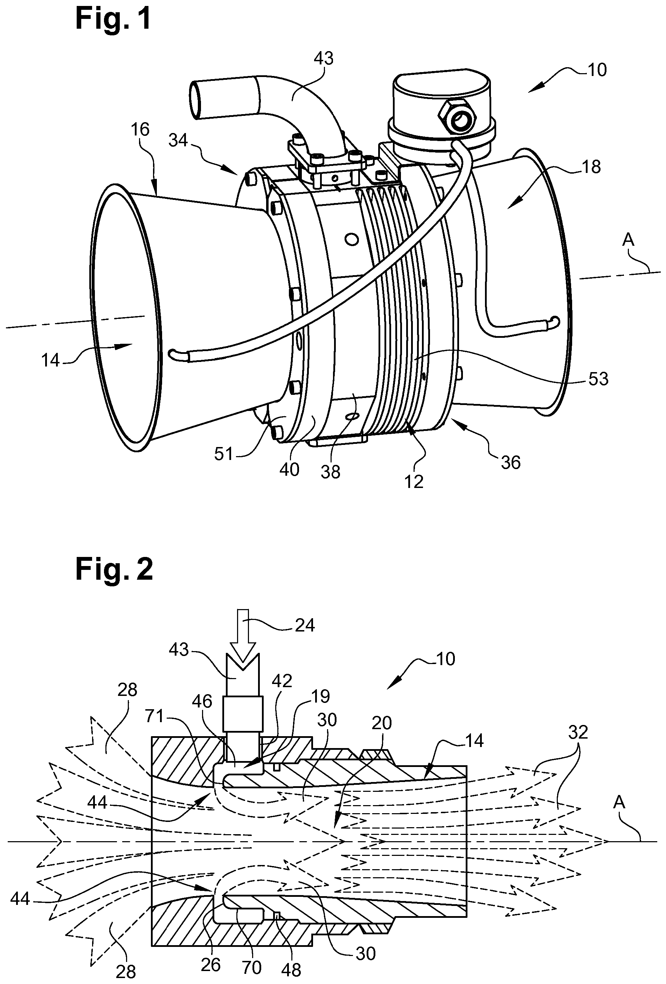

En référence à la figure 1, un amplificateur de débit 10 à effet Coanda comprend un corps d'amplificateur 12 définissant un conduit principal 14 de circulation d'air s'étendant le long d'un axe de circulation A. Par soucis de clarté, l'amplificateur de débit à effet Coanda 10 sera nommé ci-après « amplificateur de débit 10 ». Le conduit principal 14 a par exemple une longueur de plusieurs mètres, et l'amplificateur de débit 10 est placé à plus d'un mètre d'une entrée et d'une sortie de ce conduit principal 14.
L'amplificateur de débit 10 comprend également un raccord amont 16 et un raccord aval 18 connectés respectivement à une première 34 et une deuxième 36 extrémités du corps d'amplificateur 12. Les première 34 et deuxième 36 extrémités sont disposées de manière opposée le long de l'axe de circulation A. Les raccords amont 16 et aval 18 sont configurés pour raccorder l'amplificateur de débit 10 à des conduites aérauliques. De manière préférée, ces conduites aérauliques sont standards. A titre d'exemple, une conduite standard présente un diamètre interne de 200mm pour une application au convoyage de pollen.

Les raccords amont 16 et aval 18 présentent une section conique dans un plan perpendiculaire à l'axe de circulation A. Les raccords amont 16 et aval 18 comprenant chacun une extrémité proximale destinée à être fixée au corps d'amplificateur 12 et une extrémité distale destinée à être fixée à une conduite aéraulique standard. La plus petite section des raccords amont 16 et aval 18 est disposée au niveau de leur extrémité proximale destinée à être fixée au corps d'amplificateur 12. Le diamètre du conduit principal 14 est ainsi inférieur aux diamètres des conduites aérauliques auxquelles les raccords amont 16 et aval 18 sont destinés à être raccordés.

Le corps d'amplificateur 12 comprend un corps principal 38 formant en son sein le conduit principal 14. Le corps principal 38 forme une couronne de section annulaire dont la paroi interne forme le conduit principal 14. En particulier, le corps principal 38 forme une section annulaire autour de l'axe de circulation A. Le corps d'amplificateur 12 comprend également une couronne d'injection 40 disposée contre le corps principal 38 le long de l'axe de circulation A.

En référence aux figures 2 à 4, l'amplificateur de débit 10 comprend un circuit d'injection 19 de gaz moteur à l'intérieur du conduit principal 14. Ce circuit d'injection 19 est de préférence formé au moins partiellement, de manière encore préférée totalement, à l'intérieur du corps d'amplificateur 12. Selon un mode de réalisation préféré, le circuit d'injection 19 est formé au moins partiellement à l'intérieur du corps principal 38.

En référence à la figure 2 qui illustre un schéma de principe de l'amplificateur de débit 10, un flux de soufflage 32 est induit par l'amplificateur de débit 10, associant un flux de gaz moteur 30 provenant du circuit d'injection 19 et un flux d'aspiration secondaire 28. Le flux de gaz moteur primaire 30 est annulaire et disposé en périphérie du conduit principal 14 par rapport à l'axe de circulation A, au contact des parois du conduit principal 14. Le flux d'aspiration secondaire 28 est central par rapport à l'axe de circulation A et de moindre vélocité que le flux de gaz moteur primaire 30. Ainsi, cette injection d'un flux de gaz moteur primaire 30 sous forme annulaire permet d'exposer les matières fragiles convoyées essentiellement à la zone centrale de flux d'aspiration secondaire 28 de moindre vélocité. L'amplificateur de débit 10 est configuré pour induire à partir du circuit d'injection 19 le flux d'aspiration secondaire 28 d'une vitesse prédéterminée dont le ratio entre ledit flux d'aspiration secondaire 28 dans le conduit principal 14 et le flux de gaz moteur primaire 30 est supérieur ou égal à 10, de préférence supérieur ou égal à 15, de manière encore préférée supérieur ou égal à 17. Ainsi, lorsque le ratio entre ledit flux d'aspiration secondaire 28 dans le canal de convoyage 16 et le flux de gaz moteur primaire 30 est égal à 17, la quantité de gaz moteur primaire 30 est environ égale à 6% du flux de soufflage 32 en aval. Le taux de gaz moteur injecté a une grande importance sur le transport de particules fragiles car il accroît de différentiel entre la vitesse d'aspiration que l'on cherche à optimiser et la vitesse de soufflage qui ne doit pas être trop sensiblement accrue au risque de dégrader les particules transportées. L'optimisation énergétique est également un résultat attendu de l'adéquation des profils d'amplification aux vitesses de transport recherchées.

Le circuit d'injection 19 comprend une pluralité d'orifices d'alimentation 42 en gaz moteur comprimé, au moins un orifice d'injection 44 débouchant à l'intérieur du conduit principal 14 et un conduit de distribution 46 mettant en communication de fluide la pluralité d'orifices d'alimentation 42 avec le ou les orifices d'injection 44. De manière préféré, le circuit d'injection 19 ne comporte qu'un seul orifice d'injection 44 lorsqu'il comprend un seul conduit de distribution 46. De manière encore préférée, le circuit d'injection 19 comprend un nombre d'orifice d'injection 44 égal au nombre de conduit de distribution 46.

Les orifices d'alimentation 42 sont configurés pour être connectés à une source de gaz moteur comprimé 24 de manière à permettre l'injection de gaz moteur comprimé dans le conduit de distribution 46 pour être ensuite injecté dans le conduit principal 14 au travers des orifices d'injection 42. Les orifices d'alimentation 42 sont en particulier configurés pour être raccordés à un tuyau d'alimentation 43 en communication de fluide avec la source de gaz comprimé 24.

Cette source de gaz comprimé 24 peut être intégrée à l'amplificateur de débit 10 ou bien être raccordée à celui-ci. La source de gaz comprimé 24 peut être de la forme d'un compresseur relié à un réservoir de gaz pour le comprimer et l'injecter à l'intérieur du circuit d'injection 19. Ce gaz est de préférence de l'air ambiant.

Les orifices d'alimentation 42 sont formés sur une paroi extérieure du corps principal 38. Les orifices d'alimentation 42 sont de préférence orientés radialement à l'axe de circulation A pour éviter tout phénomène de tourbillon à l'intérieur du circuit d'injection 19 ainsi que dans le conduit principal 14. Une orientation tangentielle des orifices d'alimentation 42 aurait en effet tendance à générer des turbulences et tourbillons ce qui nuirait à l'uniformité et la stabilité du flux de gaz moteur. Tel qu'indiqué ci-avant, ces turbulences pourraient nuire à l'intégrité des matières fragiles transportées.

Les orifices d'alimentation 42 sont formés autour de l'axe de circulation A. De préférence, les orifices d'alimentation 42 sont équirépartis autour de l'axe de circulation A sur la paroi extérieure du corps principal 38 de manière à répartir le flux de gaz moteur dans le conduit de distribution 46. On entend par « équirépartis » le fait que le secteur angulaire séparant deux orifices d'alimentation 42 adjacents est égal à 360° divisé par le nombre total d'orifices d'alimentation 42. Ainsi, si le corps principal 38 comprend deux orifices d'alimentation 42, ceux-ci sont séparés d'un angle de 180°. Le corps principal 38 peut comprend au moins trois, au moins quatre ou encore au moins cinq orifices d'alimentation 42.

Les orifices d'alimentation 42 sont de préférence désalignés vis-à-vis de l'un ou plusieurs des orifices d'injection 44 de manière à interdire l'alimentation en gaz moteur depuis un orifice d'alimentation 42 vers un orifice d'injection selon une trajectoire rectiligne continue. En d'autres termes, un orifice d'alimentation 42 n'est pas positionné en regard d'un orifice d'injection 44 pour éviter un accès direct du gaz depuis l'orifice d'alimentation 42 vers l'orifice d'injection 44. Ainsi, les orifices d'alimentation 42 et l'au moins un orifice d'injection 44 sont de préférence décalés le long de l'axe de circulation A ou décalés angulairement autour de l'axe de circulation A. Dès lors, le conduit de distribution 46 forme une paroi de déflexion faisant face à chacune des orifices d'alimentation 42. Le flux de gaz moteur rencontre ainsi cette paroi de déflexion dès la sortie d'un orifice d'alimentation et se stabilise dans le conduit de distribution 46 avant d'être injecté au travers d'un ou plusieurs orifices d'injection 44.

Selon une configuration préférée visible en figures 2 à 4, le conduit de distribution 46 s'étend au moins partiellement le long de l'axe de circulation A. Les orifices d'alimentation 42 et l'au moins un orifice d'injection 44 sont décalés le long de l'axe de circulation A de manière à s'étendre dans des plans perpendiculaires à l'axe de circulation A distincts l'un par rapport à l'autre.

Le conduit de distribution 46 forme de préférence une cavité de distribution annulaire s'étendant le long et autour de l'axe de circulation A.

L'orifice d'injection 44 est de préférence réalisés sous la forme d'une fente 71 s'étendant au moins partiellement autour de l'axe de circulation A. La fente s'étend de préférence le long d'un secteur angulaire autour de l'axe de circulation A. De manière encore préférée, la fente 71 est circulaire et forme un orifice annulaire s'étend autour de l'axe de circulation A. Ainsi, le gaz moteur est injecté au travers de la fente sous la forme d'une lame d'air annulaire autour de l'axe de circulation A, en périphérie du conduit principal 14. La fente 71 peut être continue tout autour de l'axe de circulation A. De manière alternative, la fente 71 peut être réalisée de manière discontinue par une pluralité d'ouvertures ou de portions de fentes pour ainsi former une pluralité d'orifices d'injection 44.

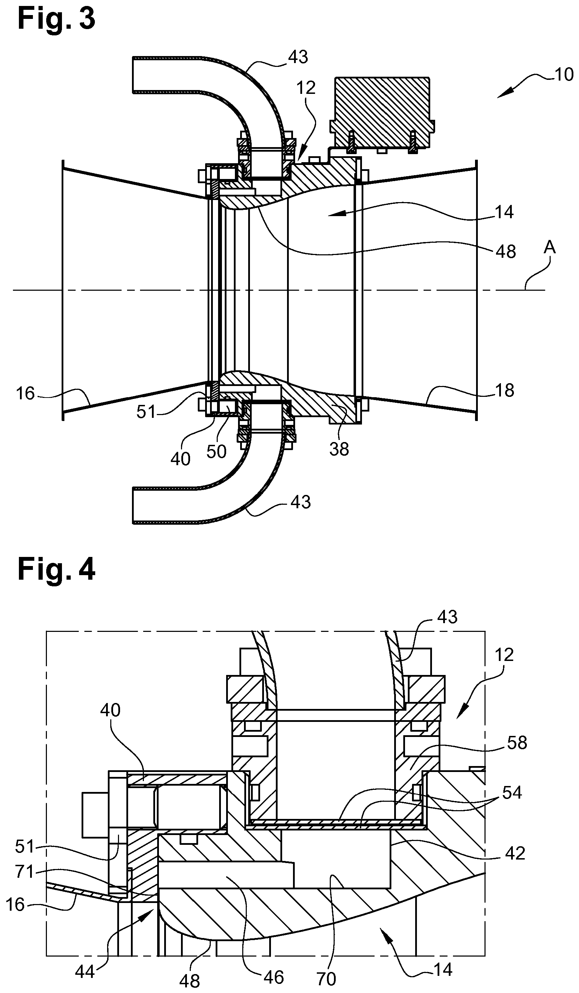

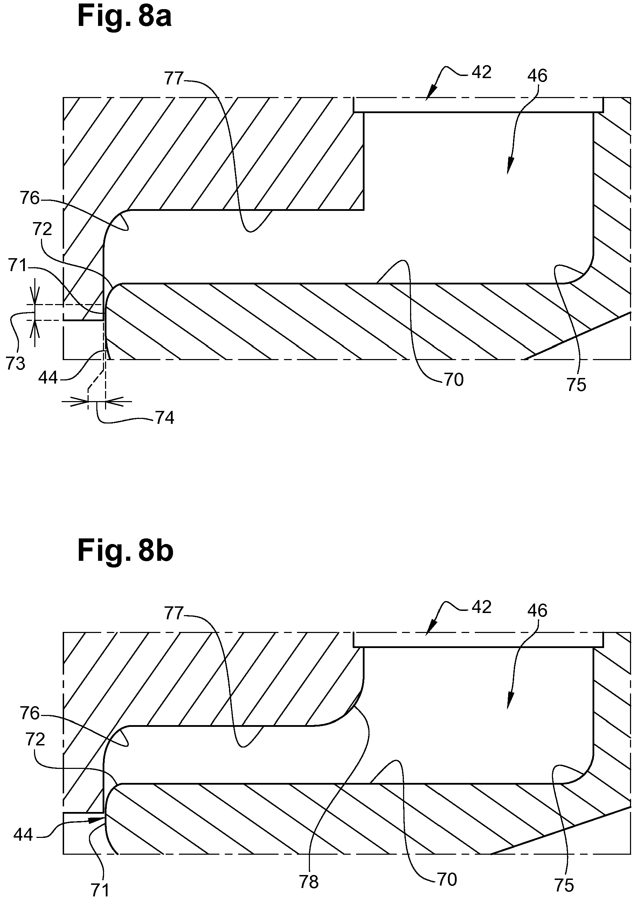

Comme cela est visible aux vues en coupes des Figures 2, 4, 8a et 8b, la fente 71 est d'une dimension radiale relativement à l'axe A, de telle sorte que l'on peut déterminer à la fois une hauteur radiale 73 de cette fente, ainsi qu'une largeur axiale 74 de la fente le long de l'axe A. La hauteur axiale de la fente 71 qui débouche par l'orifice d'injection 44 correspond à la hauteur où la fente à la même largeur axiale 74. Dans les modes de réalisation des Figures 2 et 4, la fente est plus haute radialement que celle des modes de réalisation des Figures 8a et 8b. En effet, dans les modes de réalisations des Figures 8a et 8b, la hauteur radiale 73 de cette fente 71 est limitée par la présence d'un rayon de raccordement 72 défini entre un bord de la fente et une paroi de déflection 70. Cette paroi de déflexion 70 fait face à chacun des orifices d'alimentation 42, elle est adjacente à la fente 71. Ce rayon de raccordement est par exemple de l'ordre de 0.5 à 3 mm, avantageusement compris entre 1 et 2 mm.

Comme cela est d'ailleurs visible sur les Figures 8a et 8b, il peut être prévu d'autres congés d'arêtes et rayons de raccordement dans le conduit de distribution 46. Il peut par exemple être prévu un congé d'arête 75, adjacent à la paroi de déflection 70, et en regard de l'orifice d'alimentation 42. Un autre congé d'arête 76 peut être réalisé dans la paroi 77 du corps d'amplificateur 12 qui est en regard de la fente 71, la paroi 77 et la paroi de déflection 70 définissant ensemble au moins une section du conduit de distribution 46. Un autre congé de raccordement 78, Figure 8b peut également être prévu pour adoucir la jonction entre la section définie entre les parois 77 et 70, et une section adjacente et transversale en communication avec le ou les orifices d'alimentation. Les congés d'arêtes 75, 76 et ou de raccordement 78 peuvent être compris entre 1 et 5 mm, de préférence de l'ordre de 3 mm.

Etonnamment, il a été découvert que la double alimentation augmente significativement la vitesse d'aspiration, à l'entrée du conduit principal 14, ainsi que la vitesse de soufflage au niveau de la sortie du conduit principal 14. De plus, les modifications structurelles internes du conduit de distribution, par la présence de ces congés de raccordement et congés d'arêtes améliore encore plus les performances de vitesse, et donc de débit du pollen prélevé et distribué dans une même opération.

La section de la fente le long de l'axe de circulation A peut être constante sur toute la circonférence du conduit principal 14 pour induire un débit d'air identique sur l'ensemble du périmètre de l'amplificateur de débit 10. En d'autres termes, la section de la fente peut être symétrique autour de l'axe de circulation A. De manière alternative, la section de la fente peut être variable autour de l'axe de circulation A pour induire un flux de gaz secondaire ayant une vitesse variant autour de l'axe de circulation A. La section de la fente peut ainsi être asymétrique. Cette variation de la vitesse du flux de gaz secondaire est particulièrement avantageuse pour limiter la propension naturelle du pollen à sédimenter sous l'effet de la gravité et donc améliorer le maintien en suspension du pollen. Pour cela, la section de la fente est de préférence plus importante dans sa partie supérieure que dans sa partie inférieure. En d'autres termes, la fente comporte une portion supérieure ayant une section supérieure à la section d'une portion inférieure disposée à l'opposé de la portion supérieure. Cette configuration variable de la section de la fente permet d'induire une dépression plus intense au niveau de la portion supérieure.

Le corps principal 38 comprend en outre un profil d'amplification 48 délimitant au moins partiellement l'orifice d'injection 44. Le profil d'amplification 48 forme une surface convexe configurée pour induire un effet Coanda sur le flux de gaz moteur comprimé 30 injecté au travers dudit orifice d'injection 44.

Le profil d'amplification 48 est disposé en aval au contact de l'orifice d'injection 44 par rapport au sens de déplacement des gaz et matières convoyés dans le conduit principal 14. Le profil d'amplification 48 peut être obtenu par une surface courbe de manière à optimiser l'effet Coanda. De manière alternative, le profil d'amplification 48 peut être obtenu par une pluralité de segments rectilignes pour en faciliter sa fabrication.

Le profil d'amplification 48, lorsqu'il est observé en coupe transversale, correspond de préférence à une portion d'un profil « NACA » utilisé en construction aéronautique, notamment la moitié supérieure du profil « NACA ». Ainsi, le profil d'amplification 48 comprend de préférence un bord d'attaque disposé au niveau de l'orifice d'injection 44, un extrados et un bord de fuite en direction de la deuxième extrémité 36 du corps principal 38. A titre d'exemple, le profil d'amplification 48 peut correspondre à une moitié supérieure d'un profil « NACA0030 » comprenant une cambrure de la ligne de référence (du bord d'attaque au bord de fuite) de 0 degré, une position de cambrure de 0% et une épaisseur de profil de 30% de la corde, i.e. de la distance entre le bord d'attaque et le bord de fuite.

L'effet Coanda est la propriété d'un flux de gaz ou de liquide à suivre un contour courbe adjacent comme le profil d'amplification 48 sans se détacher de celui-ci. Dans un amplificateur de débit à effet Coanda, le flux d'air moteur primaire adhère à la surface courbe sous la forme d'une couche mince d'air à forte vélocité qui s'accompagne d'une zone de dépression induisant ainsi l'entrainement de l'air ambiant à un taux multiplicatif très élevé. Le profil d'amplification 48 est configuré de manière à faire perdurer l'effet Coanda sur la plus grande longueur possible afin de maximiser la surface totale de flux d'air primaire à haute vélocité avec pour corollaire l'entraînement d'air secondaire à un taux très élevé explicitant le caractère amplificateur de débit d'un tel dispositif.

Selon une configuration préférée illustrée en figures 3 et 4, l'orifice d'injection 44 est délimité par deux parois latérales respectivement formées par le corps principal 38 et la couronne d'injection 40. En d'autres termes, l'orifice d'injection est formé par un espace ménagé entre le corps principal 38 et la couronne d'injection 40. Ainsi, une paroi d'extrémité du corps principal 38, d'une part, et une paroi d'extrémité de la couronne d'injection 40, d'autre part, forment l'orifice d'injection 44. Cette disposition permet d'obtenir un orifice d'injection 44 dont la dimension le long de l'axe de circulation A peut être précisément calibrée.

Dans cette configuration préférée, le conduit de distribution 46 débouche au niveau de cette paroi latérale du corps principal 38. La couronne d'injection 40 est conformée de manière à fermer l'extrémité débouchant dans le conduit de distribution 46 lorsque la couronne d'injection 40 est disposée contre le corps principal 38. L'un ou les deux parmi le corps principal 38 et la couronne d'injection 40 sont conformés pour maintenir un espace correspondant à la largeur de l'orifice d'injection 44 le long de l'axe de circulation A lorsqu'ils sont mis en contact l'un avec l'autre.

L'amplificateur de débit 10 comprend de préférence des moyens de réglage de la section de passage de l'orifice d'injection 44, ou au moins une portion de la pluralité d'orifices d'injection 44, de manière à réguler le débit de gaz moteur traversant ledit ou lesdits orifices d'injection 44.

Dans la configuration des figures 3 et 4, les moyens de réglage sont configurés pour régler la distance entre la couronne d'injection 40 et le corps principal 38 de manière à faire varier la section de passage du gaz moteur dudit orifice d'injection 44 ou d'au moins l'un parmi les orifices d'injection 44. La couronne d'injection 40 est ainsi mobile par rapport au corps principal 38 autour d'au moins un axe transversal à l'axe de circulation A du conduit principal 14. Les moyens de réglage sont par exemple configurés pour régler l'angle d'inclinaison de la couronne d'injection 40 par rapport audit au moins un axe transversal de manière à faire varier la section de passage de gaz moteur du ou des orifices d'injection 44 de manière asymétrique.

En pratique, la rotation de la couronne d'injection 40 fait varier la distance entre le corps principal 38 et la couronne d'injection 40 sur un secteur angulaire de l'orifice d'injection 44 ou de la pluralité d'orifices d'injection 44. Cette variation de distance entraine une variation de la section de passage du gaz moteur au travers de ce secteur angulaire et permet ainsi d'en faire varier le débit au travers de ce secteur angulaire. L'asymétrie induite par la position angulaire de la couronne d'injection 40 permet ainsi d'obtenir un débit asymétrique autour de l'axe de circulation A. On peut ainsi augmenter le débit de gaz moteur en partie supérieure de l'amplificateur de débit 10 pour compenser l'effet de la gravité sur les matières convoyées de manière à limiter tout contact entre les matières fragiles et les parois du conduit principal 14.

Pour compenser la gravité, l'asymétrie générée par la couronne d'injection 40 est réalisée en déplaçant la couronne autour d'un axe sensiblement horizontal. De manière alternative ou combinée, il est aussi possible de déplacer la couronne d'injection 40 le long d'un axe sensiblement vertical de manière à générer une asymétrie entre des secteurs angulaires latéraux. Cette asymétrie latérale pourrait par exemple permettre de déporter les particules transportées vers un côté du conduit principal 14 pour éviter un obstacle ou anticiper un virage ou une bifurcation de ce conduit en aval de l'amplificateur de débit 10 afin de limiter les risques de collision.

Les moyens de réglage permettant le déplacement de la couronne d'injection 40 comprennent par exemple une pluralité de vis de réglage 50 prenant appui sur le corps principal 38 pour régler l'écart entre la couronne d'injection 40 et le corps principal 38. Ces vis de réglage 50 sont vissées dans la couronne d'injection 40. La couronne d'injection 40 est maintenue bloquée entre les brides 51 et le corps principal 38 par l'intermédiaire de vis. La sortie et mise en appui asymétrique des vis de réglage 50 entre plusieurs secteurs angulaires de la couronne d'injection 40 permet de faire varier de manière asymétrique la distance de sépare la couronne d'injection 40 du corps principal 38.

Selon un mode de réalisation préféré, le circuit d'injection 19 comprend une pluralité de lignes de distribution indépendantes les unes des autres jusqu'à l'injection du gaz moteur à l'intérieur du conduit principal 14. Dans ce cas, l'amplificateur de débit 10 comprend une pluralité de conduits de distribution 46 indépendants les uns par rapport aux autres. L'amplificateur de débit 10 comprend également une pluralité d'orifices d'injection 44. Chaque orifice d'alimentation 42 et chaque orifice d'injection 44 appartient à une ligne de distribution de sorte qu'ils ne sont en communication de fluide qu'avec un seul conduit de distribution.

Ces lignes de distribution indépendantes permettent de former des flux de gaz moteur indépendants. Il est ainsi possible de fournir des flux de gaz moteur ayant des caractéristiques différentes, comme une pression du gaz ou une nature de gaz moteur différentes. Il pourrait en effet être envisagé de mélanger un seul flux de gaz moteur avec un additif pour fournir des caractéristiques spécifiques au niveau d'un secteur angulaire du conduit principal 14. Il est également possible de fournir des flux de gaz ayant des pressions différentes induisant des débits différents d'injection autour de l'axe de circulation A. Ces lignes de distributions distinctes permettent également l'installation de dispositif de mesure, par exemple de la pression du gaz, ou de dispositifs de sécurité.

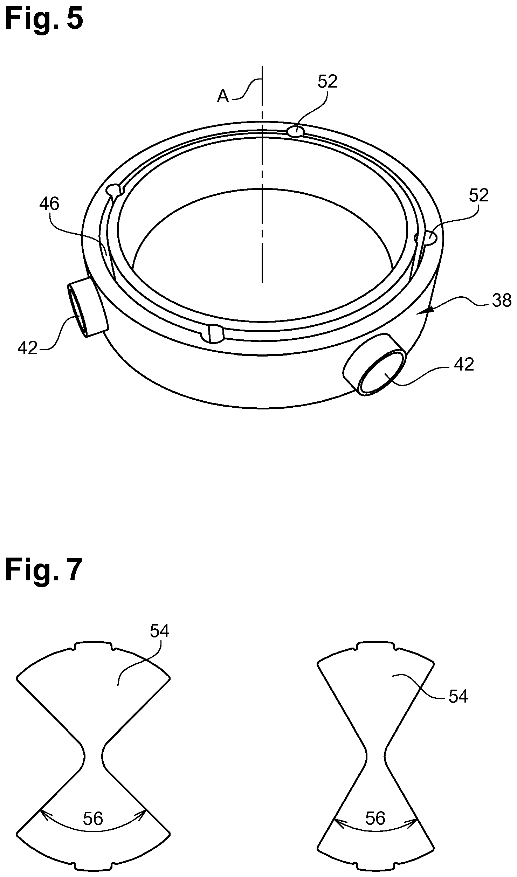

En référence à la figure 5, les conduits de distribution indépendants 46 peuvent être formés en insérant des éléments de cloisons 52 à l'intérieur d'une cavité annulaire. Les conduits de distribution 46 sont ainsi des portions d'une cavité annulaire. Ces éléments de cloisons 52 s'étendent le long de l'axe de circulation A de manière à définir des conduits de distribution 46 s'étendant le long de l'axe de circulation A. De plus, les éléments de cloison 52 peuvent être conformés pour séparer la pluralité d'orifices d'injection 44 en secteurs angulaires correspondant aux différents conduits de distribution 46. Les éléments de cloisons 52 sont de préférence disposés à l'intérieur de cette cavité annulaire de manière à segmenter la cavité annulaire en secteurs angulaires communiquant à une extrémité avec un orifice d'alimentation 42 et à une extrémité opposée avec un ou plusieurs orifices d'injection 44. L'amplificateur de débit 10 comprend de préférence autant d'orifices d'alimentation 42 que de conduits de distribution 46. Ainsi, chaque orifice d'alimentation 42 est de préférence en communication de fluide avec un seul conduit de distribution 46.

Les éléments de cloison 52 sont par exemple des cylindres, par exemple en matériau élastique, disposés entre les paroi concentriques formant la cavité annulaire. Ces cylindres sont de préférence logés à l'intérieur de logements ménagés dans les parois de la cavité annulaire. Les éléments de cloison 52 s'étendent de préférence au-delà de la paroi latérale du corps principal 38 de manière à induire une compression axiale et radiale desdits éléments de cloison 52 afin de parfaire l'étanchéité entre lignes de distribution.

De manière similaire au réglage de la section de passage des orifices d'injection 44 par le biais des moyens de réglage, les lignes de distribution peuvent être réparties autour de l'axe de circulation pour définir des secteurs angulaires autour de l'axe de circulation A de manière à pouvoir compenser la gravité ou déporter les particules convoyées vers une portion du conduit principal 14 afin d'anticiper par exemple un changement de direction ou une bifurcation. De manière alternative, il est possible de définir des zones au travers desquelles des particules de natures différentes sont convoyées. Il est ainsi possible de convoyer un premier type de matières au niveau des secteurs angulaires inférieur et supérieur et un deuxième type de matières au niveau des secteurs angulaires latéraux. La réalisation d'un flux de soufflage stable permet d'éviter qu'il y ait des échanges de matières entre ces secteurs angulaires.

Le corps principal 38 peut également comporter des reliefs externes visant à optimiser la stabilité thermique de l'amplificateur de débit 10 permettant de prévenir la formation de condensats à l'intérieur du conduit principal 14 et en particulier au niveau des orifices d'injection 44 du gaz moteur primaire qui induit par la détente partielle du gaz comprimé une réaction endothermique. La présence de condensats sur des parois exagérément refroidies est en effet très préjudiciable pour le convoyage de matières fragiles, tel que le pollen. Ces condensats pourraient souiller l'intérieur du conduit principal 14 et provoquer des adhérences ou agglutinations de pollen de sorte que le potentiel reproducteur du pollen serait diminué.

Ces reliefs peuvent être réalisés sous la forme d'ailettes 53 pourvues sur la paroi extérieure du corps principal 38. Ces reliefs ou ailettes 53 permettant d'augmenter les surfaces d'échange thermique avec l'air ambiant de manière prévenir l'apparition des condensats consécutifs à la détente des gaz primaires moteurs.

L'amplificateur de débit 10 est de préférence en fonte d'aluminium dont la bonne conductivité thermique permet d'éviter les points froids générateurs de condensation. De préférence, l'amplificateur de débit 10 est réalisé dans un matériau ayant une conductivité thermique égale ou supérieure à 150 W.m-1.K-1.

Selon un mode de réalisation, l'amplificateur de débit 10 peut également comprendre des moyens de variation du débit de gaz au travers du circuit d'injection 19. Ces moyens de variation du débit de gaz peuvent être configuré pour faire varier la pression du gaz moteur à l'intérieur des lignes de distribution ou bien pour faire varier la section de passage du gaz au niveau des orifices d'alimentation 42. Cette deuxième alternative est par exemple illustrée en figures 4, 6 et 7 dans laquelle les moyens de variation du débit de gaz comprennent au moins un élément réducteur de section 54 disposé au travers d'un orifice d'alimentation 42. Cet élément réducteur de section 54 présente une paroi latérale destinée à faire obstacle au flux de gaz moteur traversant l'orifice d'alimentation 42. Cette paroi latérale de l'élément réducteur de section 54 est calibrée de sorte que la section de passage restant disponible pour le passage du gaz moteur est connue. Cet élément réducteur de section 54 est intégré de manière amovible dans l'orifice d'alimentation 42 pour permettre de choisir la perte de charge subie par le gaz moteur et donc son débit de circulation au travers de l'orifice d'alimentation 42. De manière préférée, l'amplificateur de débit 10 appartient à un kit d'amplification de débit comprenant l'amplificateur de débit 10 et un set d'éléments réducteurs de section 54 ayant des sections d'obstruction calibrées et différentes. L'utilisateur peut ainsi choisir de mettre des éléments réducteurs de section 54 de différents calibrages au travers des orifices d'alimentation 42 de manière à obtenir des débits différents dans les lignes de distribution.

En référence à la figure 7, l'élément réducteur de section 54 peut être de la forme d'un papillon comprenant une partie centrale et deux secteurs angulaires s'étendant dans des directions opposées à partir de cette portion centrale. La dimension angulaire 56 de chacun des secteurs angulaires est ensuite prédéterminée pour correspondre à un certain niveau de réduction de section. A titre d'exemple, l'élément réducteur de section 54 disposé à gauche sur la figure 7 présente une dimension angulaire 56 égale à 60°. De manière similaire, l'élément réducteur de section 54 disposé à droite sur la figure 7 présente une dimension angulaire 56 égale à 90°.

Selon une configuration préférée illustrée en figure 3 et 4, les moyens de variation utilisent une combinaison de deux éléments réducteurs de section 54 superposés l'un sur l'autre au travers de l'orifice d'alimentation 42. Ces deux éléments réducteurs de section 54 sont mobiles en rotation l'un par rapport à l'autre de manière à pouvoir faire varier la section de passage disponible pour le gaz moteur. A titre d'exemple, un premier élément réducteur de section 54 est solidarisé au corps principal 38 et un deuxième élément réducteur de section 54 est solidarisé à une pièce mobile en rotation, ici une bague de réglage 58. Ainsi, la bague de réglage 58 est montée de manière à pouvoir être mobile en rotation par rapport au corps principal 38. Ainsi, une rotation de la bague de réglage 58 permet une rotation du deuxième élément réducteur de section 54 et donc une variation de la section de passage disponible pour le gaz moteur. La bague de réglage du débit 58 est disposée entre le tuyau d'alimentation 43 et le corps principal 38.

Cette configuration utilisant une pluralité d'éléments réducteurs de section 54 permet un réglage continu du débit de gaz moteur traversant l'orifice d'alimentation 42. Ce réglage est ainsi plus flexibilité et plus précis. En effet, le réglage des débits de gaz moteur en basses pressions est très sensible et demande un dispositif continu de réglage pour être pleinement satisfaisant. L'adoption d'un jeu complet d'éléments réducteurs de section 54, par exemple d'un angle 56 de 30, 60 et 90°, permet une plage d'obstruction du conduit de distribution 46 depuis 60° par l'adoption d'un jeu de réducteurs de section 54 30° précisément alignés et/ou superposés et jusqu'à une fermeture totale de 360° si nécessaire par l'utilisation d'un jeu de deux éléments réducteurs de section 54 de 90°décalés de 90°.

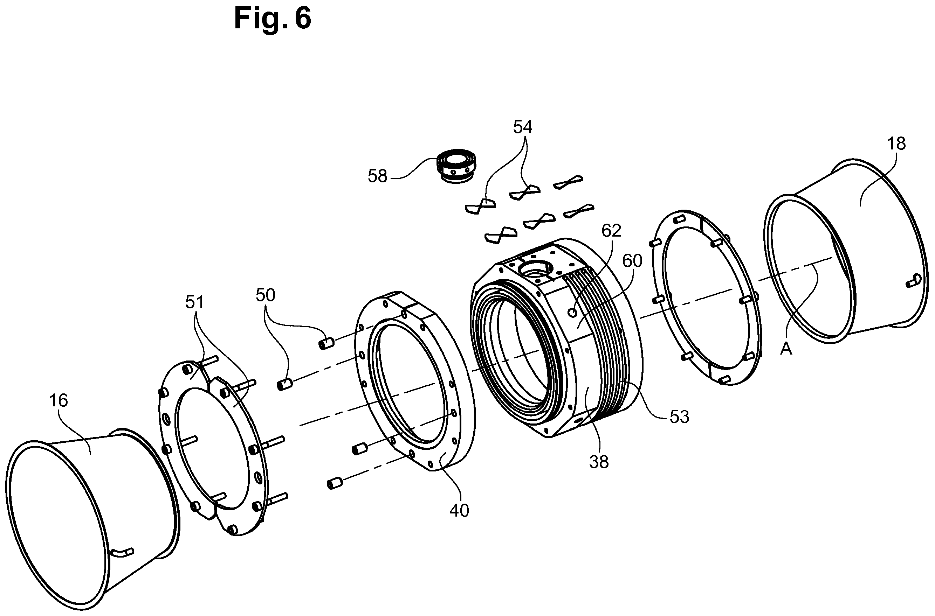

En référence à la figure 6, le corps principal 38 peut également comporter au moins une surface d'appui 60 plane formée sur sa paroi extérieure. Cette surface d'appui 60 permet l'intégration de l'amplificateur de débit 10 au sein d'un dispositif aéraulique. Un perçage non débouchant 62 peut ainsi être prévu à proximité de ou sur cette surface d'appui 60 pour fixer l'amplificateur de débit à une structure. Cette surface d'appui 60 permet d'éviter l'utilisation de pièces d'interface qui alourdiraient le dispositif aéraulique. La surface d'appui 60 permet également une meilleure continuité électrique avec la structure de manière à évacuer l'électricité statique générée par le frottement triboélectrique des matières convoyées.

Il est également proposé un appareil aéraulique comprenant un amplificateur de débit 10 tel que décrit avant, une conduite amont à connecter au raccord amont 16 et une conduite aval à connecter au raccord aval 18 de l'amplificateur de débit 10.

L'appareil aéraulique est par exemple un appareil aéraulique pour la pollinisation d'au moins une plante receveuse à partir du pollen capté sur au moins une plante donneuse. L'appareil aéraulique comprend en outre un organe de captation du pollen depuis ladite au moins une plante donneuse, un organe de diffusion du pollen sur au moins une plante receveuse et un canal de convoyage du pollen capté depuis l'organe de captation vers l'organe de diffusion. L'amplificateur de débit 10 est disposé dans le canal de convoyage pour mouvoir le pollen au travers de ce canal de convoyage.

## Revendications

1. Amplificateur de débit (10) à effet Coanda pour induire un flux de gaz amplifié, comportant :
- un conduit principal (14) de circulation d'air,
- au moins un orifice d'injection (44) débouchant dans le conduit principal (14),
- une pluralité d'orifices d'alimentation (42) en gaz moteur comprimé chacun configuré pour être connecté à une source de gaz (24) moteur comprimé pour alimenter ledit au moins un orifice d'injection (44) en gaz moteur comprimé,
- au moins un conduit de distribution (46) reliant ladite pluralité d'orifices d'alimentation (42) audit au moins un orifice d'injection (44),
- un profil d'amplification (48) délimitant au moins partiellement ledit au moins un orifice d'injection (44) et formant une surface convexe configurée pour induire un effet Coanda sur un flux de gaz moteur comprimé injecté au travers dudit au moins un orifice d'injection (44),
- le conduit principal de circulation d'air s'étendant le long d'un axe de circulation (A), ledit au moins un conduit de distribution formant une cavité de distribution annulaire s'étendant le long et autour de l'axe de circulation, ledit au moins un orifice d'injection formant une fente s'étendant au moins partiellement autour de l'axe de circulation, et
- une dimension radiale de la fente étant limitée par la présence d'un rayon de raccordement (71) présentée par une paroi de déflexion (70) faisant face à chacun des orifices d'alimentation, cette paroi de déflexion étant adjacente à la fente débouchant sur l'orifice d'injection (44).

2. Amplificateur de débit selon la revendication 1, dans lequel ledit au moins un orifice d'injection est formé par une cavité d'injection annulaire s'étendant autour de l'axe de circulation et radialement par rapport à cet axe de circulation.

3. Amplificateur de débit selon l'une quelconque des revendications 1 ou 2, dans lequel les orifices d'alimentation sont orientés transversalement à l'axe de circulation, l'amplificateur de débit comprenant en outre au moins une paroi de déflexion (70) faisant face à chacun des orifices d'alimentation.

4. Amplificateur de débit selon l'une quelconque des revendications précédentes, comprenant une pluralité de conduits de distribution indépendants les uns par rapport aux autres et une pluralité d'orifices d'injection (44), chaque conduit de distribution (46) s'étendant entre au moins l'un parmi la pluralité d'orifices d'alimentation (42) et au moins l'un parmi la pluralité d'orifices d'injection (44) de manière à pouvoir injecter au travers de la pluralité d'orifices d'injection (44) des flux de gaz moteur comprimé distincts.

5. Amplificateur de débit selon la revendication 3 en combinaison avec la revendication 2, dans lequel ladite pluralité de conduits de distribution est formée par la cavité de distribution annulaire, l'amplificateur de débit comprenant en outre au moins deux éléments de cloison (52) permettant de compartimenter la cavité de distribution pour former au moins deux conduits de distribution indépendants.

6. Amplificateur de débit selon l'une quelconque des revendications précédentes, comprenant en outre des moyens de réglage d'une section de passage du gaz moteur dudit au moins un orifice d'injection de manière à réguler le débit de gaz moteur traversant ledit au moins un orifice d'injection.

7. Amplificateur de débit selon la revendication 3 en combinaison avec la revendication précédente, dans lequel les moyens de réglage sont configurés pour régler distinctement la section de passage de gaz moteur d'au moins deux orifices d'injection (44) communiquant avec des conduits de distribution indépendants de manière à pouvoir injecter au travers desdits au moins deux orifices des flux de gaz moteur comprimé de débits différents.

8. Amplificateur de débit selon la revendication précédente, dans lequel la pluralité d'orifices d'injection comprend au moins un premier et au moins un deuxième orifices d'injection destinés à être disposés respectivement en partie inférieure et en partie supérieure du conduit principal de circulation d'air de manière à pouvoir induire en parties inférieure et supérieure un débit d'air amplifié différent.

9. Amplificateur de débit selon la revendication précédente, dans lequel les moyens de réglage sont configurés pour régler distinctement la section de passage de gaz moteur d'au moins quatre orifices d'injection communiquant avec des conduits de distribution indépendants, la pluralité d'orifices d'injection comprenant en outre au moins un troisième et au moins un quatrième orifices d'injection destinés à être disposés respectivement au niveau de parties latérales opposées du conduit principal de circulation d'air.

10. Amplificateur de débit selon l'une quelconque des revendications précédentes, comprenant :
- un corps (38) dans lequel sont formés ledit conduit principal de circulation d'air, la pluralité d'orifices d'alimentation, ledit au moins un conduit de distribution, le profil d'amplification et une première portion dudit au moins un orifice d'injection,
- une couronne d'injection (40) formant une deuxième portion dudit au moins un orifice d'injection, la couronne d'injection étant configurée pour être disposée en regard du corps, les première et deuxième portions dudit au moins un orifice d'injection se faisant face, la distance séparant les première et deuxième portions dudit au moins un orifice d'injection définissant une section de passage de gaz moteur au travers dudit au moins un orifice d'injection.

11. Amplificateur de débit selon la revendication précédente en combinaison avec la revendication 5, dans lequel les moyens de réglage sont configurés pour régler la distance entre la couronne d'injection et le corps de manière à faire varier la section de passage du gaz moteur dudit au moins un orifice d'injection.

12. Amplificateur de débit selon la revendication précédente, dans lequel la couronne est mobile par rapport au corps autour d'au moins un axe transversal à un axe de circulation du conduit principal de circulation, les moyens de réglage étant configurés pour régler l'angle d'inclinaison de la couronne par rapport audit au moins un axe transversal de manière à faire varier la section de passage de gaz moteur dudit au moins un orifice d'injection de manière asymétrique.

13. Appareil aéraulique pour la pollinisation d'au moins une plante receveuse à partir du pollen capté sur au moins une plante donneuse, comprenant :
- un organe de captation du pollen depuis ladite au moins une plante donneuse,
- au moins un organe de diffusion du pollen sur au moins une plante receveuse,
- un canal de convoyage du pollen capté depuis l'organe de captation vers le ou les organes de diffusion, et
- au moins un amplificateur de débit selon l'une quelconque des revendications précédentes.

14. Utilisation d'un amplificateur de débit selon l'une quelconque des revendications 1 à 12 pour l'amplification d'un flux d'air comprenant des particules présentant une vitesse de sédimentation prédéterminée, dans laquelle l'amplificateur de débit à effet Coanda induit un flux d'air à l'intérieur du conduit principal de circulation dont la vitesse est supérieure à la vitesse de sédimentation prédéterminée.

15. Utilisation selon la revendication 14, dans laquelle la vitesse du flux d'air induit à l'intérieur du conduit principal de circulation est égale ou inférieure à 10m.s-1, de préférence égale ou inférieure à 5m.s-1.

## Patentansprüche

1. Coanda-Effekt-Durchflussverstärker (10) zum Induzieren eines verstärkten Gasstroms, umfassend:
- eine Hauptleitung (14) zur Zirkulation von Luft,
- mindestens eine Einspritzöffnung (44), die in der Hauptleitung (14) mündet,
- eine Vielzahl von Öffnungen (42) zur Zuführung von komprimiertem Treibgas, von denen jede dazu konfiguriert ist, mit einer Quelle (24) von komprimiertem Treibgas verbunden zu sein, um der mindestens einen Einspritzöffnung (44) komprimiertes Treibgas zuzuführen,
- mindestens eine Verteilungsleitung (46), die die Vielzahl von Zuführöffnungen (42) mit der mindestens einen Einspritzöffnung (44) verbindet,
- ein Verstärkungsprofil (48), das die mindestens eine Einspritzöffnung (44) mindestens teilweise abgrenzt und eine konvexe Oberfläche bildet, die dazu konfiguriert ist, an einem komprimierten Treibgasstrom, der durch die mindestens eine Einspritzöffnung (44) eingespritzt wird, einen Coanda-Effekt zu induzieren,
- wobei sich die Hauptleitung zur Zirkulation von Luft entlang einer Zirkulationsachse (A) erstreckt, wobei die mindestens eine Verteilungsleitung einen ringförmigen Verteilungshohlraum bildet, der sich entlang der und um die Zirkulationsachse erstreckt, wobei die mindestens eine Einspritzöffnung einen Spalt bildet, der sich mindestens teilweise um die Zirkulationsachse erstreckt, und
- wobei eine radiale Abmessung des Spalts durch das Vorhandensein eines Übergangsradius (71), der durch eine Ablenkwand (70), die jeder der Zuführöffnungen gegenüberliegt, bereitgestellt wird, begrenzt wird, wobei diese Ablenkwand an den Spalt, der an der Einspritzöffnung (44) mündet, angrenzt.

2. Durchflussverstärker nach Anspruch 1, wobei die mindestens eine Einspritzöffnung durch einen ringförmigen Einspritzhohlraum gebildet ist, der sich um die Zirkulationsachse und in Bezug auf diese Zirkulationsachse radial erstreckt.

3. Durchflussverstärker nach einem der Ansprüche 1 oder 2, wobei die Zuführöffnungen quer zu der Zirkulationsachse eingerichtet sind, wobei der Durchflussverstärker ferner mindestens eine Ablenkwand (70) beinhaltet, die jeder der Zuführöffnungen gegenüberliegt.

4. Durchflussverstärker nach einem der vorhergehenden Ansprüche, beinhaltend eine Vielzahl von Verteilungsleitungen, die untereinander unabhängig sind, und eine Vielzahl von Einspritzöffnungen (44), wobei sich jede Verteilungsleitung (46) zwischen mindestens einer der Vielzahl von Zuführöffnungen (42) und mindestens einer der Vielzahl von Einspritzöffnungen (44) erstreckt, um verschiedene komprimierte Treibgasströme durch die Vielzahl von Einspritzöffnungen (44) einspritzen zu können.

5. Durchflussverstärker nach Anspruch 3 in Kombination mit Anspruch 2, wobei die Vielzahl von Verteilungsleitungen durch den ringförmigen Verteilungshohlraum gebildet ist, wobei der Durchflussverstärker ferner mindestens zwei Trennwandelemente (52) beinhaltet, die es gestatten, den Verteilungshohlraum zu unterteilen, um mindestens zwei unabhängige Verteilungsleitungen zu bilden.

6. Durchflussverstärker nach einem der vorhergehenden Ansprüche, ferner beinhaltend Mittel zum Einstellen eines Durchgangsquerschnitts des Treibgases der mindestens einen Einspritzöffnung, um den Treibgasdurchfluss, der durch die mindestens eine Einspritzöffnung hindurchgeht, zu regulieren.

7. Durchflussverstärker nach Anspruch 3 in Kombination mit dem vorhergehenden Anspruch, wobei die Einstellmittel dazu konfiguriert sind, den Treibgasdurchgangsquerschnitt von mindestens zwei Einspritzöffnungen (44), die mit unabhängigen Verteilungsleitungen kommunizieren, gesondert einzustellen, um durch die mindestens zwei Öffnungen komprimierte Treibgasströme mit unterschiedlichen Durchflüssen einspritzen zu können.

8. Durchflussverstärker nach dem vorhergehenden Anspruch, wobei die Vielzahl von Einspritzöffnungen mindestens eine erste und mindestens eine zweite Einspritzöffnung beinhaltet, die dazu bestimmt sind, jeweils im unteren Teil und im oberen Teil der Hauptleitung zur Zirkulation von Luft angeordnet zu sein, um im unteren und oberen Teil einen unterschiedlichen verstärkten Luftdurchfluss induzieren zu können.

9. Durchflussverstärker nach dem vorhergehenden Anspruch, wobei die Einstellmittel dazu konfiguriert sind, den Treibgasdurchgangsquerschnitt von mindestens vier Einspritzöffnungen, die mit unabhängigen Verteilungsleitungen kommunizieren, gesondert einzustellen, wobei die Vielzahl von Einspritzöffnungen ferner mindestens eine dritte und mindestens eine vierte Einspritzöffnung beinhaltet, die dazu bestimmt sind, jeweils an gegenüberliegenden seitlichen Teilen der Hauptleitung zur Zirkulation von Luft angeordnet zu sein.

10. Durchflussverstärker nach einem der vorhergehenden Ansprüche, beinhaltend:
- einen Körper (38), in dem die Hauptleitung zur Zirkulation von Luft, die Vielzahl von Zuführöffnungen, die mindestens eine Verteilungsleitung, das Verstärkungsprofil und ein erster Abschnitt der mindestens einen Einspritzöffnung gebildet sind,
- einen Einspritzkranz (40), der einen zweiten Abschnitt der mindestens einen Einspritzöffnung bildet, wobei der Einspritzkranz dazu konfiguriert ist, gegenüber dem Körper angeordnet zu sein, wobei sich der erste und der zweite Abschnitt der mindestens einen Einspritzöffnung gegenüberliegen, wobei der Abstand, der den ersten und den zweiten Abschnitt der mindestens einen Einspritzöffnung trennt, einen Durchgangsquerschnitt von Treibgas durch die mindestens eine Einspritzöffnung definiert.

11. Durchflussverstärker nach dem vorhergehenden Anspruch in Kombination mit Anspruch 5, wobei die Einstellmittel dazu konfiguriert sind, den Abstand zwischen dem Einspritzkranz und dem Körper einzustellen, um den Durchgangsquerschnitt des Treibgases der mindestens einen Einspritzöffnung variieren zu lassen.

12. Durchflussverstärker nach dem vorhergehenden Anspruch, wobei der Kranz in Bezug auf den Körper um mindestens eine Achse, die zu einer Zirkulationsachse der Hauptzirkulationsleitung quer verläuft, beweglich ist, wobei die Einstellmittel dazu konfiguriert sind, den Neigungswinkel des Kranzes in Bezug auf die mindestens eine Querachse einzustellen, um den Treibgasdurchgangsquerschnitt der mindestens einen Einspritzöffnung auf asymmetrische Weise variieren zu lassen.

13. Lufteinrichtung zur Bestäubung mindestens einer Empfängerpflanze mit Hilfe von Pollen, der an einer Spenderpflanze gewonnen wird, beinhaltend:
- ein Organ zum Gewinnen des Pollens von der mindestens einen Spenderpflanze,
- mindestens ein Organ zur Verbreitung des Pollens auf mindestens einer Empfängerpflanze,
- einen Kanal zum Befördern des gewonnenen Pollens von dem Gewinnungsorgan zu dem oder den Verbreitungsorganen und
- mindesten einen Durchflussverstärker nach einem der vorhergehenden Ansprüche.

14. Verwendung eines Durchflussverstärkers nach einem der Ansprüche 1 bis 12 zur Verstärkung eines Luftstroms, der Partikel beinhaltet, die eine vorbestimmte Sedimentationsgeschwindigkeit aufweisen, wobei der Coanda-Effekt-Durchflussverstärker innerhalb der Hauptzirkulationsleitung einen Luftstrom induziert, dessen Geschwindigkeit größer als die vorbestimmte Sedimentationsgeschwindigkeit ist.

15. Verwendung nach Anspruch 14, wobei die Geschwindigkeit des innerhalb der Hauptzirkulationsleitung induzierten Luftstroms gleich oder kleiner als 10m.s-1, vorzugsweise gleich oder kleiner als 5m.s-1, ist.

## Claims

1. Coanda effect flow amplifier (10) for bringing about an amplified gas flow, having:
- a main air circulation duct (14),
- at least one injection orifice (44) opening into the main duct (14),
- a plurality of orifices (42) for feeding compressed driving gas, each being configured to be connected to a source (24) of compressed driving gas in order to feed said at least one injection orifice (44) with compressed driving gas,
- at least one distribution duct (46) connecting said plurality of feed orifices (42) to said at least one injection orifice (44),
- an amplification profile (48) at least partially delimiting said at least one injection orifice (44) and forming a convex surface configured to bring about a Coanda effect in a flow of compressed driving gas injected through said at least one injection orifice (44),
- the main air circulation duct extending along a circulation axis (A), said at least one distribution duct forming an annular distribution cavity extending along and around the circulation axis, said at least one injection orifice forming a slot extending at least partially around the circulation axis, and
- a radial dimension of the slot being limited by the presence of a connecting radius (71) presented by a deflecting wall (70) facing each of the feed orifices, this deflecting wall being adjacent to the slot opening onto the injection orifice (44).

2. Flow amplifier according to Claim 1, wherein said at least one injection orifice is formed by an annular injection cavity extending around the circulation axis and radially with respect to this circulation axis.

3. Flow amplifier according to either one of Claims 1 and 2, wherein the feed orifices are oriented transversely to the circulation axis, the flow amplifier also comprising at least one deflecting wall (70) facing each of the feed orifices.

4. Flow amplifier according to any one of the preceding claims, comprising a plurality of mutually independent distribution ducts and a plurality of injection orifices (44), each distribution duct (46) extending between at least one of the plurality of feed orifices (42) and at least one of the plurality of injection orifices (44) so that it is possible to inject separate flows of compressed driving gas through the plurality of injection orifices (44).

5. Flow amplifier according to Claim 3 in combination with Claim 2, wherein said plurality of distribution ducts is formed by the annular distribution cavity, the flow amplifier also comprising at least two partition elements (52) for compartmentalizing the distribution cavity so as to form at least two independent distribution ducts.

6. Flow amplifier according to any one of the preceding claims, also comprising means for adjusting a flow cross section for the driving gas of said at least one injection orifice so as to regulate the flow rate of driving gas passing through said at least one injection orifice.

7. Flow amplifier according to Claim 3 in combination with the preceding claim, wherein the adjusting means are configured to separately adjust the flow cross section for driving gas of at least two injection orifices (44) communicating with independent distribution ducts so that it is possible to inject flows of compressed driving gas with different flow rates through said at least two orifices.

8. Flow amplifier according to the preceding claim, wherein the plurality of injection orifices comprises at least one first and at least one second injection orifice, which are intended to be arranged respectively in the lower part and in the upper part of the main air circulation duct so that it is possible to bring about a different amplified air flow rate in the lower and upper parts.

9. Flow amplifier according to the preceding claim, wherein the adjusting means are configured to separately adjust the flow cross section for driving gas of at least four injection orifices communicating with independent distribution ducts, the plurality of injection orifices also comprising at least one third and at least one fourth injection orifice, which are intended to be arranged respectively at opposite lateral parts of the main air circulation duct.

10. Flow amplifier according to any one of the preceding claims, comprising:
- a body (38) in which said main air circulation duct, the plurality of feed orifices, said at least one distribution duct, the amplification profile and a first portion of said at least one injection orifice are formed,
- an injection ring (40) forming a second portion of said at least one injection orifice, the injection ring being configured to be arranged facing the body, the first and second portions of said at least one injection orifice facing one another, the distance separating the first and second portions of said at least one injection orifice defining a flow cross section for driving gas through said at least one injection orifice.

11. Flow amplifier according to the preceding claim in combination with Claim 5, wherein the adjusting means are configured to adjust the distance between the injection ring and the body so as to vary the flow cross section for driving gas of said at least one injection orifice.

12. Flow amplifier according to the preceding claim, wherein the ring is movable with respect to the body about at least one axis transverse to a circulation axis of the main circulation duct, the adjusting means being configured to adjust the inclination angle of the ring with respect to said at least one transverse axis so as to vary the flow cross section for driving gas of said at least one injection orifice asymmetrically.

13. Aeraulic apparatus for pollinating at least one receiver plant with pollen collected from at least one donor plant, comprising:
- a member for collecting the pollen from said at least one donor plant,
- at least one member for diffusing the pollen over at least one receiver plant,
- a channel for conveying the pollen collected from the collecting member to the one or more diffusing members, and
- at least one flow amplifier according to any one of the preceding claims.

14. Use of a flow amplifier according to any one of Claims 1 to 12 to amplify an air flow comprising particles exhibiting a predetermined sedimentation rate, wherein the Coanda effect flow amplifier brings about an air flow inside the main circulation duct, the speed of which is higher than the predetermined sedimentation rate.

15. Use according to Claim 14, wherein the speed of the air flow brought about inside the main circulation duct is less than or equal to 10 m.s-1, preferably less than or equal to 5 m.s-1.
